(19) Europäisches Patentamt
European Patent Office
Office européen des brevets

(11) Veröffentlichungsnummer: **0 073 960**
**A1**

# (12) EUROPÄISCHE PATENTANMELDUNG

(21) Anmeldenummer: **82107420.0**

(22) Anmeldetag: **16.08.82**

(51) Int. Cl.³: **C 07 D 233/52**
A 61 K 31/415

(30) Priorität: **27.08.81 DE 3133887**

(43) Veröffentlichungstag der Anmeldung:
**16.03.83 Patentblatt 83/11**

(84) Benannte Vertragsstaaten:
**CH DE FR GB LI**

(71) Anmelder: **BAYER AG**
**Zentralbereich Patente, Marken und Lizenzen**
**D-5090 Leverkusen 1, Bayerwerk(DE)**

(72) Erfinder: **Behner, Otto, Dr.**
**In den Birken 83**
**D-5600 Wuppertal 1(DE)**

(72) Erfinder: **Stendel, Wilhelm, Dr.**
**In den Birken 55**
**D-5600 Wuppertal 1(DE)**

(72) Erfinder: **Andrews, Peter, Dr.**
**Gellertweg 2**
**D-5600 Wuppertal 1(DE)**

(54) **2-Arylhydrazino-2-imidazoline, deren Acylderivate, Verfahren zu ihrer Herstellung und ihre Verwendung zur Bekämpfung von Endo- und Ektoparasiten.**

(57) Imidazolinverbindungen der Formel

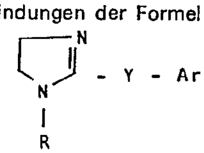 (1)

in der

Y für 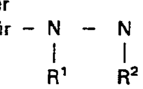 steht und

$R^1$ und $R^2$ Wasserstoff oder Acyl bedeuten,

Ar einen mit identischen Substituenten disubstituierten oder mit einem weiteren Ring anellierten Phenylrest bedeutet und

R für Wasserstoff, $C_1$-$C_4$-Alkyl oder einen Acylrest steht,

Verfahren zu ihrer Herstellung und ihre Verwendung zur Bekämpfung von Ektoparasiten und Endoparasiten.

EP 0 073 960 A1

0073960

BAYER AKTIENGESELLSCHAFT 5090 Leverkusen, Bayerwerk

Zentralbereich
Patente, Marken und Lizenzen Ad/m-c

# 2-Arylhydrazino-2-imidazoline, deren Acylderivate, Verfahren zu ihrer Herstellung und ihre Verwendung zur Bekämpfung von Endo- und Ektoparasiten

Die Erfindung betrifft neue 2-Arylhydrazino-2-imidazoline und ihre Acylderivate, Verfahren zur ihrer Herstellung und ihre Verwendung zur Bekämpfung von Ektoparasiten und Endoparasiten.

Im Benzolring substituierte 2-Phenylhydrazino-2-imidazoline sind bekannt (z.B. US-PS 4,117,146, US-PS 3,480,630). Diesen Verbindungen wird magensaftsekretionshemmende, antihypertensive oder antidepressive Wirkung zugeschrieben. Weiterhin sind bekannt 2-(subst.)Aryl-2-alkylhydrazino-2-imidazoline mit antihypertensiver Wirkung (Südafrik. Patentanmeldung 67/5654). Über eine evtl. antiparasitäre Wirkung ist in der zitierten Literatur nichts geschrieben.

Bekannt sind weiterhin in der Hydrazinbrücke acylierte, im Benzolring mono- oder in 2,4-, 2,6- oder 3,4-Stellung disubstituierte 2-(2-Phenylhydrazino)-2-imidazoline mit Wirkung gegen Zecken. (DE-OSen 2 041 732, 2 041 733 und 2 041 734).

Es wurde nun gefunden, daß die neuen Imidazoline der Formel I

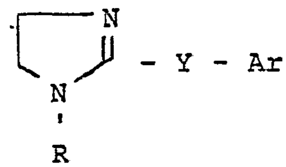

(I)

in der

Y für einen Rest $-\underset{R^1}{N}-\underset{R^2}{N}-$ steht und

$R^1$ und $R^2$ Wasserstoff oder Acyl bedeuten und

Ar einen mit identischen Substituenten disubstituierten oder mit einem weiteren Ring anellierten Phenylrest bedeutet und

R für Wasserstoff, einen $C_1$-$C_4$-Alkylrest oder einen Acylrest steht, bzw. deren Salze

hervorragend geeignet sind zur Bekämpfung von tierischen Ektoparasiten, insbesondere aus der Klasse der Akariden, sowie von Endoparasiten.

Bei den Acylresten handelt es sich bevorzugt um gegebenenfalls substituierte $C_1$-$C_6$-Alkanoylreste, gegebenenfalls substituierte $C_2$-$C_6$-Alkenoylreste, gegebenenfalls substituierte $C_7$-$C_{10}$-Aroylreste, $C_2$-$C_7$-Alkoxycarbonyl- oder Carbamoylreste, $C_1$-$C_6$-Alkylsulfonylreste oder gegebenenfalls durch $C_1$-$C_2$-Alkylgruppen, Nitrogruppen oder Halogenatome ein- oder mehrfach substituierte $C_6$-$C_{10}$-Arylsulfonylreste.

Bei den Acylresten handelt es sich insbesondere um Formyl-, Acetyl-, Trichloracetyl-, Trifluoracetyl-, Propionyl-, Crotonyl-, Benzoyl-, Chlorbenzoyl-, Ethoxycarbonyl-, Methoxycarbonyl-, N-Methylcarbamoyl-, N-Phenylcarbamoyl-, Methansulfonyl-, Ethansulfonyl-, Benzolsulfonyl-, Chlorbenzolsulfonyl-, Toluolsulfonyl-, Nitrobenzolsulfonyl- oder Naphthalinsulfonylreste.

Bei dem Rest Ar handelt es sich um durch $C_1$-$C_3$-Alkyl-, $C_1$-$C_3$-Alkoxy- oder Trifluormethylgruppen oder vorzugsweise in 2,3-Stellung disubstituiertes Phenyl, um Naphthyl, Tetrahydronaphthyl oder Indanyl-Reste.

Bei dem Rest Ar handelt es sich vorzugsweise um einen 2,3-Dimethylphenyl-, 1-Naphthyl-, 5,6,7,8-Tetrahydro-1-naphthyl- oder 4-Indanylrest.

Bei dem Rest R handelt es sich um Wasserstoff, einen Alkylrest mit 1 bis 4 C-Atomen oder einen Acylrest nach der oben stehenden Definition, vorzugsweise um Wasserstoff, eine Methyl-, Methansulfonyl- oder Benzolsulfonylgruppe.

Weiterhin wurde gefunden, daß man die erfindungsgemäßen Verbindungen der Formel I, in der Y für die Gruppe -NH-NH- steht und R Wasserstoff oder Alkyl bedeutet, erhält, wenn man

a) Arylhydrazine der Formel II

$$Ar - NH - NH_2 \qquad (II)$$

mit Imidazolinen der Formel III

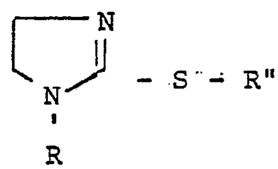

(III)

in der

R die oben angegebene Bedeutung hat und

R" für einen gegebenenfalls substituierten Alkylrest steht,

vorteilhaft in Gegenwart einer starken Säure umsetzt, oder

b) Thiosemicarbazide der Formel IV oder Isothiosemicarbazide der Formel V, in denen Ar und R" die oben angegebene Bedeutung haben,

$$Ar - NH - NH - CS - NH_2 \qquad (IV)$$

$$Ar - NH - NH - \underset{\substack{| \\ S-R''}}{C} = NH \qquad (V)$$

gegebenenfalls in Gegenwart einer starken Säure mit Ethylendiamin umsetzt, oder

c) Arylhydrazine der Formel II mit dem Reaktionsprodukt aus Ethylenharnstoff und Phosphorpentachlorid, von dem angenommen wird, daß es die Struktur von N,N'-Bis-(2-oxoimidazolidin-1-yl)-phosphorsäurechlorid hat, unter Bedingungen zur Reaktion bringt, wie sie analog in Bull. Soc. Chim. France 1 961,2114 beschrieben sind, oder

d) N-Arylamino-N'-nitroguanidine der Formel VI,

$$\begin{array}{l} Ar - NH - NH - C = NH \\ \qquad\qquad\qquad\quad\; | \\ \qquad\qquad\qquad\quad NH - NO_2 \end{array} \qquad (VI)$$

in der Ar die oben angegebene Bedeutung hat, und die analog zu J.A.C.S. 72, 5343 (1950) aus Arylhydrazinen der Formel II und N-Methyl-N-nitroso-N'-nitroguanidin hergestellt werden können, mit Ethylendiamin oder einem Salz davon umsetzt.

Die Acylierung der Verbindungen der Formel I, in der Ar die oben angegebene Bedeutung hat und $R^1$ und $R^2$ Wasserstoff bedeutet und R für Wasserstoff oder Alkyl steht, zu Verbindungen der Formel I, in der $R^1$ oder $R^2$ ein Acylrest der oben angegebenen Bedeutung ist und R für Wasserstoff, Alkyl oder einen Acylrest der oben angegebenen Bedeutung steht, wird auf an sich bekannte Weise durchgeführt, beispielsweise indem man die nicht acylierten Verbindungen in einem geeigneten Lösungsmittel oder Lösungsmittelgemisch in Gegenwart eines Säurefängers mit Sulfonsäurehalogeniden, Carbonsäurehalogeniden oder -anhydriden oder Chlorameisensäurehalogeniden oder -anhydriden zur Reaktion bringt oder mit Isocyanaten in Abwesenheit einer Base reagieren läßt.

Als Säurefänger eingesetzte Basen können sein z.B. Alkali- oder Erdalkali-hydroxide, -carbonate oder -hydrogencarbonate oder tertiäre Amine wie Triethylamin oder Pyridin.

Bevorzugt betrifft die Erfindung die im folgenden beispielhaft erwähnten Verbindungen:

2-[2-(2,3-Dimethylphenyl)hydrazino]-2-imidazolin,
[2,3-Dimethoxy-3-methylphenyl)-hydrazino]-2-imidazolin
[2,3-Dimethoxy-3-trifluormethylphenyl)hydrazino]-2-imidazolin,
[2,3-Dichlor-2-isopropylphenyl)hydrazino]-2-imidazolin,
2-[2-(1-Naphthyl)hydrazino]-2-imidazolin
2-[2-(5,6,7,8-Tetrahydro-2-naphthyl)hydrazino]-2-imidazolin,
2-[2-(4-Indanyl)hydrazino]-2-imidazolin,
2-[2-(2,3-Dimethylphenyl)hydrazino]-1-methyl-2-imidazolin,
2-[2-(1-Naphthyl)hydrazino]-1-methyl-2-imidazolin,
2-[2-(5,6,7,8-Tetrahydro-1-naphthyl)hydrazino]-1-methyl-2-imidazolin,
2-[2-(2,3-Dimethylphenyl)-1-(oder 2)-phenylsulfonylhydrazino]-2-imidazolin,
2-[2-(2,3-Dimethylphenyl)-1-methylsulfonylhydrazino]-1-methylsulfonyl-2-imidazolin,
2-[2-(2,3-Dimethylphenyl)-1-(oder 2)-(4-chlorphenylsulfonyl)-hydrazino]-2-imidazolin,

2-/2-(1-Naphthyl)-1-(oder 2)-phenylsulfonylhydrazino7-2-imidazolin
2-/2-(2,3-Dimethylphenyl)-1,2-bis-(phenylsulfonyl)hydrazino7-1-methyl-2-imidazolin,
2-/2-(5,6,7,8-Tetrahydro-1-naphthyl)-1(oder 2)-(4-nitrophenylsulfonyl)hydrazino7-2-imidazolin,
2-/2-(2,3-Dimethylphenyl-1(oder 2)-(4-tolylsulfonyl)-hydrazino7-2-imidazolin,
2-/2-(2,3-Dimethylphenyl)-1(oder 2)-acetylhydrazino7-2-imidazolin,
2-/2-(2,3-Dimethylphenyl)-1(oder 2)-benzoylhydrazino7-2-imidazolin
2-/2-(2,3-Dimethylphenyl)-1-(oder 2)-ethoxycarbonyl-hydrazino7-2-imidazolin,
2-/2-(2,3-Dimethylphenyl)-1(oder 2)-N-methylcarbamoyl-hydrazino7-1-N-methylcarbamoyl-2-imidazolin.

Die neuen Wirkstoffe der allgemeinen Formel sowie gegebenenfalls ihre Salze weisen starke acarizide Wirkung auf, besonders gegen Acariden, die als tierische Ektoparasiten domestizierte Tiere, wie Rinder, Schafe und Kaninchen befallen. Gleichzeitig haben die erfindungsgemäßen Verbindungen nur eine geringe Warmblütertoxizität. Sie eignen sich daher gut zur Bekämpfung von tierischen Ektoparasiten aus der Klasse der Acariden. Darüber hinaus besitzen sie jedoch auch Wirkung gegen andere Acariden sowie gegen Insekten.

Als wirtschaftlich wichtige Ektoparasiten, die besonders

in tropischen und subtropischen Ländern eine große Rolle spielen, seien genannt: die australische und südamerikanische Rinderzecke Boophilus microplus, die südafrikanische Rinderzecke Boophilus decoloratus, beide aus der Familie der Ixodidae, sowie Rinder- und Schafzecken der Genera Rhipicephalus, Amblyomma, Hyalomma.

Im Laufe der Zeit sind insbesondere Zecken gegen die als Bekämpfungsmittel bisher verwendeten Phosphorsäureester und Carbamate resistent geworden, so daß der Bekämpfungserfolg in vielen Gebieten in wachsendem Maße infrage gestellt wird. Zur Sicherung einer wirtschaftlichen Viehhaltung in den Befallsgebieten besteht ein dringender Bedarf an Mitteln, mit denen alle Entwicklungsstadien, also Larven, Nymphen, Metanymphen und Adulti auch resistenter Stämme, beispielsweise des Genus Boophilus sicher bekämpft werden können. In hohem Maße gegen die bisher verwendeten Phosphorsäureester resistent sind beispielsweise in Australien der Mackay-Stamm, der Biarra-Stamm und der Mt-Alford-Stamm von Boophilus microplus.

Die erfindungsgemäßen Wirkstoffe sind sowohl gegen die normalempfindlichen, als auch gegen die resistenten Stämme, z.B. von Boophilus, gleich gut wirksam. Sie wirken in üblicher Applikation am Wirtstier sowohl direkt auf alle am Tier parasitierenden Formen als auch stark ovizid auf die adulten Formen, so daß der Vermehrungscyclus der Zecken sowohl in der parasitischen Phase auf dem Tier als auch in der nicht parasitären Phase unterbrochen wird. Die Eiablage wird unterbunden,

die Entwicklung und das Schlüpfen inhibiert. Hervorzuheben sind insbesondere der schnell eintretende erregende Effekt auf alle parasitierenden Formen, die ihre Saughaltung aufgeben, in unphysiologischer Weise auf dem Wirtstier umherwandern, abfallen und schließlich sterben (detaching effect) sowie insbesondere auch die gute Wirkung gegen die erfahrungsgemäß schwer bekämpfbaren Metanymphenstadien.

Ferner wirken sie in der gleichen Art auf alle Entwicklungsstadien mehrwirtiger Zecken wie z.B. Amblyomma spp., Hyalomma spp., Rhipicephalus spp., Ixodes spp., Hämaphysalis spp., Dermacentor spp.

Ein detaching effect zeigt sich auch bei Insekten, beispielsweise bei Läusen, wie Hämatopinus spp.

Die neuen Verbindungen der Formel I sind darüber hinaus auch gegen Endoparasiten wirksam. Sie wirken vor allem gegen Magen- und Darmnematoden der Wiederkäuer und Fleischfresser, auch solche, die gegen die gebräuchlichen Benzimidazolanthelmintika resistent und damit nicht mehr ausreichend therapierbar sind.

Sie wirken vorzugsweise gegen Haemonchus in besonders niedriger Dosierung.

Die Wirkung wurde im Tierversuch nach oraler, subcutaner und dermaler Applikation bei stark mit Parasiten befallenen Versuchstieren geprüft. Die angewendeten Dosierungen wurden sehr gut von den Versuchstieren vertragen.

Die Verbindung wirkt nicht nur nach oraler oder parenteraler Verabreichung, sondern auch gut nach dermaler Applikation (Pour-On, Spot-On). Damit werden Verabreichungsweisen möglich, die bei den im Handel befindlichen nur nach oraler Gabe wirkenden Benzimidazol-Anthelmintika nicht möglich sind, andererseits aber für den Anwender vorteilhaft sind wie z.B. die dermale oder subcutane Behandlung.

Die neuen Verbindungen können als Anthelmintika sowohl in der Human- als auch in der Veterinärmedizin verwendet werden. Sie können in bekannter Weise in die üblichen Formulierungen übergeführt werden.

Je nach der vorgesehenen Applikationsform können die neuen Wirkstoffe in die praxisüblichen Formulierungen übergeführt werden, wie beispielsweise Lösungen, Emulsionen, Suspensionen, Pulver, Pasten und Granulate. Diese werden in bekannter Weise hergestellt, z.B. durch Vermischen der Wirkstoffe mit Streckmitteln, d.h., flüssigen Lösungsmitteln und/oder Trägerstoffen, gegebenenfalls unter Mitverwendung von oberflächenaktiven Mitteln, also Emulgier- und/oder Dispergiermitteln, wobei z.B. im Falle der Verwendung von Wasser als Streckmittel gegebenenfalls organische Lösungsmittel als Hilfslösungsmittel verwendet werden können.

Als Lösungsmittel kommen z.B. infrage: Aromaten (z.B. Xylol, Benzol, Orthodichlorbenzol, Trichlorbenzol), Paraffine (z.B. Erdölfraktionen), Alkohole (z.B. Methanol, Ethanol, Isopropanol, Butanol), stark polare

Lösungsmittel wie Dimethylformamid, N-Methyl-pyrrolidon, Dimethylsulfoxid sowie auch Wasser.

Als feste Trägerstoffe seien genannt: natürliche Gesteinsmehle (z.B. Kaoline, Tonerden, Talkum, Kreide) synthetische anorganische Trägerstoffe (z.B. hochdisperse Kieselsäure, Silikate); als Emulgiermittel: sowohl nichtionogene als auch anionische oder kationische Emulgatoren wie z.B. Polyoxethylen-Fettsäureester, Polyoxethylen-Fettalkoholether, z.B. Alkyl-arylpolyglykolether; Alkylsulfonate und Arylsulfonate, quartäre Ammoniumsalze mit längeren Alkylresten. Als Dispergiermittel seien genannt: Lignin, Sulfitablaugen und Methylcellulose.

Die Formulierungen enthalten im allgemeinen zwischen 0,1 und 95 Gew.-% Wirkstoff, vorzugsweise zwischen 0,5 bis 90 Gew.-%. Die Anwendungskonzentrationen werden aus den Formulierungen (s.o.) durch Verdünnen mit Wasser hergestellt. Sie können, je nach der Anwendungsform, in einem großen Bereich variiert werden und liegen zwischen 1 und 50.000 ppm (g/g), vorzugsweise zwischen 5 und 500 ppm.

Die Applikation erfolgt in üblicher Weise, z.B. durch Besprühen (spray), Gießen (pour on), Vernebeln oder durch Bad (dip).

Den Formulierungen oder den anwendungsfertigen Lösungen können noch sonstige Hilfsmittel oder Wirkstoffe, wie Desinfektionsmittel oder speziell geeignete Insektizide, zugemischt werden.

Die wäßrigen Lösungen bzw. Emulsionen der erfindungsgemäßen Wirkstoffe besitzen unter Praxisbedingungen eine gute Stabilität, so daß die gebrauchsfertigen Anwendungsformen auch bei längerem Stehen und in einem pH-Bereich von 7 - 9 drei Monate und länger wirksam bleiben.

Beispiel 1

2-/2-(2,3-Dimethylphenyl)-hydrazino/-2-imidazolin

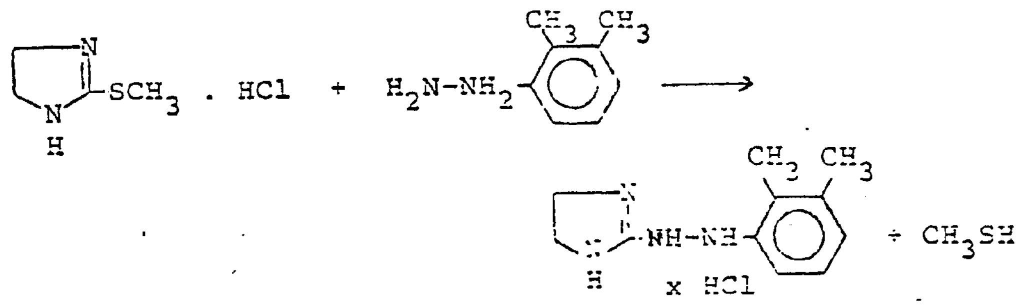

18,3 g (0,12 Mol) 2-Methylthio-2-imidazolin-hydrochlorid und 16,4 g (0,12 Mol) 2,3-Dimethylphenylhydrazin werden in 60 ml Xylol unter Rühren langsam erhitzt und 2 Stunden bei 120 - 130°C gehalten, bis die Methylmercaptan-Entwicklung beendet ist. Man saugt nach dem Abkühlen ab und kristallisiert aus Ethanol um.
Fp. 260 - 262°C (Z.) (Hydrochlorid), 15,3 g (53 % d.Th.)

Beispiel 2

2-/2-(2,3-Dimethylphenyl)-hydrazino/-1-methyl-2-imidazolin

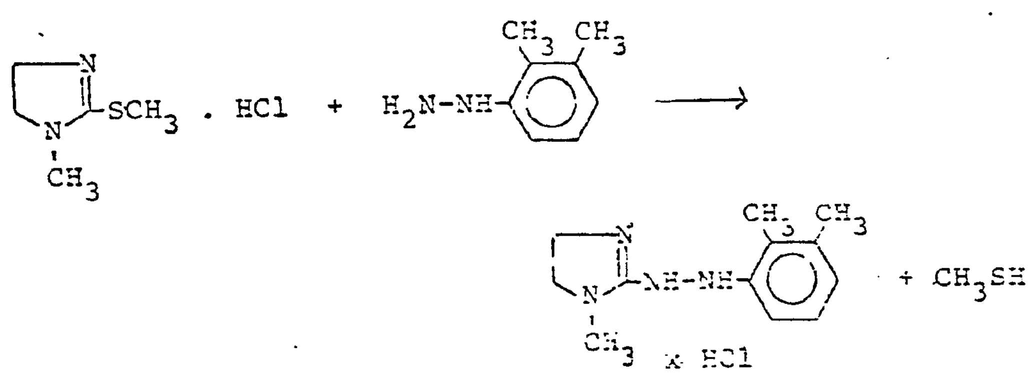



BAD ORIGINAL

Ein Gemisch aus 5,3 g (0,0309 Mol) 2,3-Dimethylphenylhydrazin und 5,0 g (0,03 Mol) 1-Methyl-2-methylthio-2-imidazolin-hydrochlorid in 45 ml Isopropanol wird unter Stickstoff 24 Stunden unter R.F. gerührt. Man saugt die heiße Lösung durch Kieselgur ab, dampft im Vakuum zur Trockne ein und verreibt den Rückstand mit 70 ml Ether. Das Hydrochlorid (6,8 g ≙ 89 % d.Th.) schmilzt unscharf zwischen 60°C und 74°C. Das daraus auf übliche Weise in Wasser mit Dinatriumnaphthalin-1,5-disulfonat hergestellte Hemi-naphthalin-1,5-disulfonat hat bei 142 - 144°C einen Umwandlungspunkt und ist bei 260°C noch nicht geschmolzen.

Beispiel 3

2-/2-(1-Naphthyl)-hydrazino/-2-imidazolin
Analog Beispiel 2 aus 2-Methylthio-2-imidazolin-hydrochlorid und 1-Naphthylhydrazin, Fp. des Hydrochlorids unscharf bis 203°C; das Hemi-naphthalin-1,5-disulfonat ist bei 250°C noch nicht geschmolzen.

Beispiel 4

1-Methyl-2-/2-(1-naphthyl)-hydrazino/-2-imidazolin
Analog Beispiel 2 aus 1-Methyl-2-methylthio-2-imidazolin und 1-Naphthylhydrazin. Das Hemi-naphthalin-1,5-disulfonat ist bei 260°C noch nicht geschmolzen.

Beispiel 5

2-/2-(5,6,7,8-Tetrahydro-1-naphthyl)-hydrazino7-2-imidazolin

Analog Beispiel 2 aus 2-Methylthio-2-imidazolin-hydrochlorid und Tetrahydronaphthylhydrazin. Hydrochlorid Fp. 234-236° (Z.)

Beispiel 6

1-Methyl-2-/2-(5,6,7,8-tetrahydro-1-naphthyl)-hydrazino7-2-imidazolin

Analog Beispiel 2 aus 1-Methyl-2-methylthio-2-imidazolin-hydrochlorid und Tetrahydronaphthylhydrazin. Hemi-naphthalin-1,5-disulfonat Fp. 233-240°C (Z.)

Beispiel 7

2-/2-(2,3-Dimethylphenyl)-1-(oder 2)-phenylsulfonyl-hydrazino7-2-imidazolin

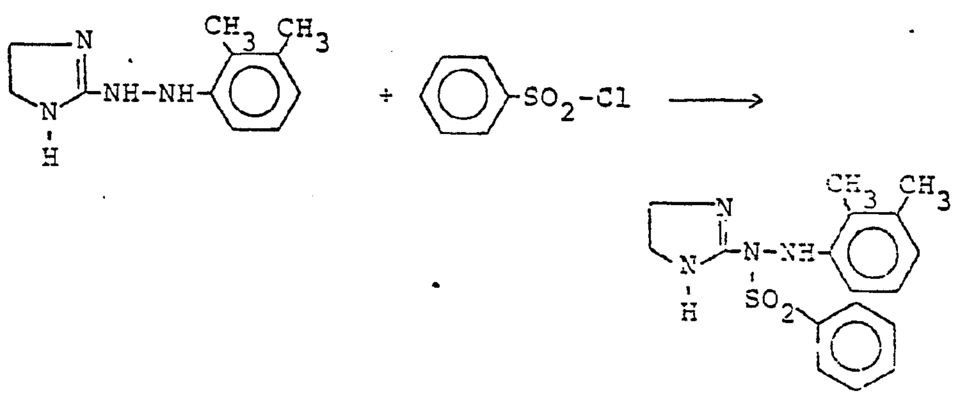

Ein Gemisch aus 2,4 g (0,01 Mol) 2-[2-(2,3-Dimethyl-phenyl)-hydrazino]-2-imidazolin-hydrochlorid in 20 ml Methylenchlorid und 1,2 g (0,03 Mol) Natriumhydroxid in 5 ml Wasser wird unter Rühren innerhalb von 15 Min. versetzt mit einer Lösung von 1,94 g (10 % Überschuß) Benzolsulfonylchlorid in 10 ml Methylenchlorid. Man rührt 1 Stunde bei R.T. nach, verdünnt mit etwas Wasser, trennt die Phasen, trocknet die org. Phase über Kalium-carbonat und dampft zur Trockne ein. Das rote, harzige Produkt wird mit 15 ml Ethanol kurz aufgekocht, wobei es durchkristallisiert. Nach dem Abkühlen und Absaugen farblose Kristalle, 2,6 g (75,5 % d.Th.), Fp, 143 - 144°C (Z.).

Beispiel 8

2-[1-(oder 2)-(4-Chlorphenylsulfonyl)-2-(2,3-dimethyl-phenyl)-hydrazino]-2-imidazolin
Analog Beispiel 7 aus 2-[2-(2,3-Dimethylphenyl)-hydrazino]-2-imidazolin-hydrochlorid und 4-Chlorbenzolsulfonylchlorid. Fp. 137 - 140°C (Z.).

Beispiel 9

2-[2-(2,3-Dimethylphenyl-1-methylsulfonyl)-hydrazino]-1-methylsulfonyl-2-imidazolin

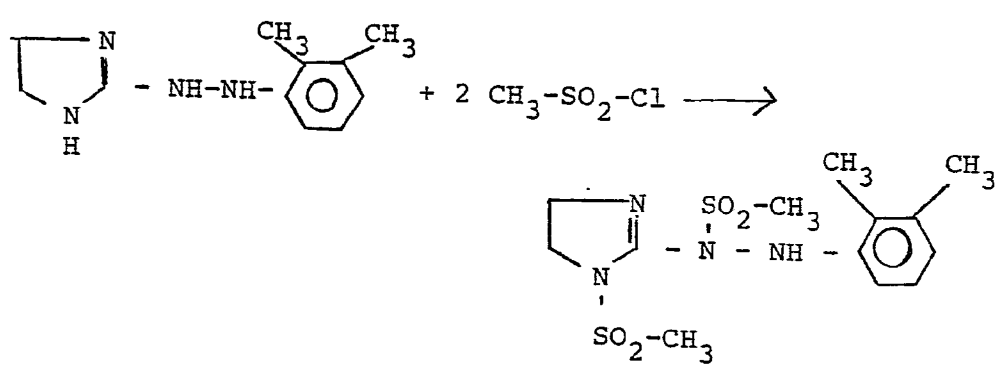

Ein Gemisch aus 4,8 g (0,02 Mol 2-[2-(2,3-Dimethylphenyl)-hydrazino]-2-imidazolin-hydrochlorid, 6,67 g (0,066 Mol) Triethylamin und 40 ml Methylenchlorid wird bei 5° unter Rühren mit 5,0 g (0,044 Mol) Methansulfonylchlorid in 20 ml Methylenchlorid versetzt. Man rührt 1 Stunde ohne Kühlung nach, verdünnt mit 100 ml trockenem Ether, saugt ab, dampft das Filtrat im Vakuum ein und kocht den Eindampfrückstand kurz mit 10 ml Isopropanol auf. Fp. 127-131°C (Z.), Ausbeute 4,1 g (69,5 % d.Th.).

<u>Beispiel 10</u>

2-[2-(2,3-Dimethylphenyl)-1,2-bis-(phenylsulfonyl)-hydrazino]-1-methyl-2-imidazolin
Analog Beispiel 9 aus 2-[2-(2,3-Dimethylphenyl)-hydrazino]-1-methyl-2-imidazolin-hydrochlorid und Benzolsulfochlorid. Fp. 168 - 169°C (Z.).

Zeckentest

Lösungsmittel: 35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

In diese Wirkstoffzubereitungen werden adulte, vollgesogene Zeckenweibchen der Art Boophilus microplus (resistent) eine Minute lang getaucht. Nach dem Tauchen von je 10 weiblichen Exemplaren überführt man diese in Petrischalen, deren Boden mit einer entsprechend großen Filterscheibe belegt ist.

Nach 20 Tagen wird die Wirksamkeit der Wirkstoffzubereitung bestimmt durch Ermittlung der Hemmung der Eiablage gegenüber unbehandelten Kontrollzecken. Die Wirkung drückt man in Prozent aus, wobei 100 % bedeutet, daß keine Eier mehr abgelegt wurden und 0 % besagt, daß die Zecken Eier in normaler Menge ablegten.

Untersuchter Wirkstoff, geprüfte Konzentration, getestete Parasiten und erhaltene Befunde gehen aus der folgenden Tabelle 1 hervor.

Tabelle 1

In-vitro-Test auf eiablagehemmende Wirkung
an Zecken (Boophilus microplus, Biarra-Stamm)

| Wirkstoff | 100 % | 50 % |
|---|---|---|
| | Hemmung bei einer Wirkstoffkonzentration in ppm a.i.von | |
| 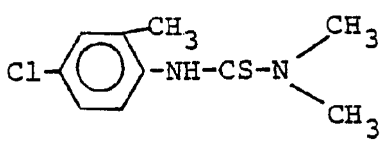<br>Chloromethiuron<br>(bekanntes Vergleichspräparat) | - | >5.000 |
| 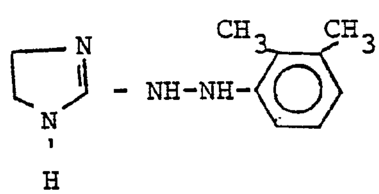 | 3.000 | |

Magen- und Darmwurmtest/Schaf

Experimentell mit Haemonchus contortus infizierte Schafe wurden nach Ablauf der Präpatenzzeit der Parasiten behandelt. Die Wirkstoffmenge wurde als reiner Wirkstoff in Gelatinekapseln oral appliziert oder in einem geeigneten Lösungsmittel gelöst und subcutan oder dermal appliziert.

Der Wirksungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren können (Dosis effectiva). Geprüfte Wirkstoffe und wirksame Dosierungen (dosis effectiva minima) sind aus der nachfolgenden Tabelle ersichtlich.

| Wirkstoff | Applikations-weise | Dosis effectiva minima (Red.≥90% in mg/kg) |
|---|---|---|
| 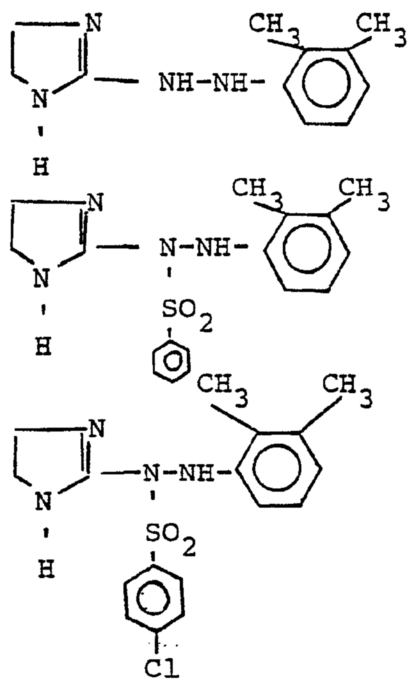 | per os | 1 |
| | per os | 1 |
| | per os | 1 |
| 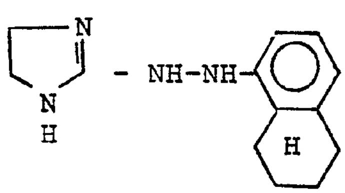 | per os | 25 |

Patentansprüche:

1. Imidazolinverbindungen der Formel

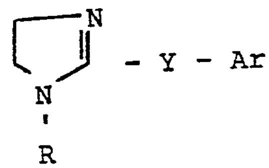

(I)

in der

Y für $-N(R^1)-N(R^2)-$ steht und

$R^1$ und $R^2$ Wasserstoff oder Acyl bedeuten,

Ar einen mit identischen Substituenten disubstituierten oder mit einem weiteren Ring anellierten Phenylrest bedeutet und

R für Wasserstoff, $C_1$-$C_4$-Alkyl oder einen Acylrest steht.

2. Imidazolinderivate nach Anspruch 1, dadurch gekennzeichnet, daß einer der Reste $R^1$ oder $R^2$ für einen gegebenenfalls substituierten $C_1$-$C_6$-Alkanoyl-, $C_7$-$C_{10}$-Aroyl-, $C_2$-$C_6$-Alkoxycarbonyl- oder Carbamoylrest, einen $C_1$-$C_6$-Alkylsulfonylrest oder einen gegebenenfalls durch $C_1$-$C_2$-Alkylgruppen, Nitrogruppen oder Halogenatome ein- oder mehrfach substituierten $C_6$-$C_{10}$-Arylsulfonylrest steht und Ar durch $C_1$-$C_3$-Alkyl, $C_1$-$C_3$-Alkoxy oder $-CF_3$ disubstituiertes oder mit einem weiteren Ring anelliertes Phenyl steht.

3. Imidazolinderivate nach Anspruch 2, dadurch gekennzeichnet, daß der Rest Ar in 2- und 3-Stellung substituiert oder anelliert ist.

4. Verfahren zur Herstellung von Imidazolinverbindungen der Formel

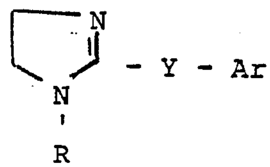

(I)

in der

Y für $-N(R^1)-N(R^2)-$ steht und

$R^1$ und $R^2$ Wasserstoff bedeutet,

Ar einen mit identischen Substituenten disubstituierten oder mit einem weiteren Ring anellierten Phenylrest bedeutet und

R für H oder $C_1$-$C_4$-Alkyl steht,

dadurch gekennzeichnet, daß man

a) Arylhydrazine der Formel

$$Ar - NH - NH_2 \qquad (II)$$

mit Imidazolinen der Formel

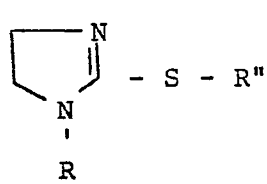

(III)

in der

R die oben angegebene Bedeutung hat und

R" für einen gegebenenfalls substituierten Alkylrest steht,

gegebenenfalls in Gegenwart einer starken Säure umsetzt, oder

b) Thiosemicarbazide der Formel IV oder Isothiosemicarbazide der Formel V, in denen Ar und R" die oben angegebenen Bedeutung haben,

$$Ar - NH - NH - CS - NH_2 \qquad (IV)$$

$$Ar - NH - NH - \underset{\substack{|\\ S-R''}}{C} = NH \qquad (V)$$

gegebenenfalls in Gegenwart einer starken Säure mit Ethylendiamin umsetzt, oder

c) Arylhydrazine der Formel II mit dem Reaktionsprodukt aus Ethylenharnstoff und Phosphorpentachlorid umsetzt, oder

d) N-Arylamino-N'-nitro-guanidine der Formel

$$Ar - NH - NH - \underset{\substack{|\\ NH - NO_2}}{C} = NH \qquad (VI)$$

in der Ar die oben angegebene Bedeutung hat, mit Ethylendiamin oder einem Salz davon umsetzt.

5. Verfahren zur Herstellung von Imidazolinderivaten der Formel

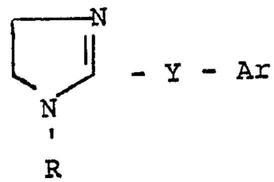

in der

Y für $-\underset{R^1}{N}-\underset{R^2}{N}-$ steht und

$R^1$ und $R^2$ Wasserstoff oder Acyl bedeuten mit der Maßgabe, daß mindestens einer der Substituenten $R^1$ oder $R^2$ für Acyl steht,

Ar einen mit identischen Substituenten disubstituierten oder mit einem weiteren Ring anellierten Phenylrest bedeutet und

R für H, einen $C_1$-$C_4$-Alkyl- oder Acylrest steht,

dadurch gekennzeichnet, daß man Imidazolinderivate nach Anspruch 4 mit Säurehalogeniden, -anhydriden oder mit Isocyanaten zur Reaktion bringt.

6. Endo- und/oder ektoparasitizides Mittel, gekennzeichnet durch einen Gehalt an mindestens einer Imidazolinverbindung gemäß Anspruch 1.

7. Anthelmintisches Mittel nach Anspruch 6.

8. Zeckenwirksames Mittel nach Anspruch 6.

9. Verfahren zur Herstellung von endo- und/oder ektoparasitiziden Mitteln, dadurch gekennzeichnet, daß man Imidazolinverbindungen gemäß Anspruch 1 mit inerten, nichttoxischen, pharmazeutisch geeigneten Trägerstoffen vermischt.

10. Verwendung von Imidazolinverbindungen gemäß Anspruch 1 in endo- und/oder ektoparasitiziden Mitteln und bei der Bekämpfung von Endo- und/oder Ektoparasiten.

Europäisches Patentamt

# EUROPÄISCHER RECHERCHENBERICHT

0073960
Nummer der Anmeldung

EP 82 10 7420.0

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.3) |
|---|---|---|---|
| D,X | DE - A - 2 041 733 (BAYER AG)<br>* Ansprüche 1 bis 4; Seite 2, Zeilen 19 bis 26; Seite 12, Zeilen 19, 20 * | 1,2,5, 6,8,9 | C 07 D 233/52<br>A 61 K 31/415 |
| X | DE - A - 1 670 124 (C.H. BOEHRINGER SOHN)<br>* Ansprüche 1, 2; Seite 1, Formel I, Zeilen 10 bis 17; Seite 4, Zeilen 13 bis 17 * | 1,4 | |
| D,A | DE - A - 2 041 732 (BAYER AG)<br>* Ansprüche 1 bis 4; Seite 2, Zeilen 18 bis 24 * | 1,2,5, 6,8,9 | RECHERCHIERTE SACHGEBIETE (Int. Cl.3) |
| D,A | US - A - 4 117 146 (A.H. ABDALLAH et al.)<br>* Anspruch 6; Spalte 4, Formel, Tabelle 1 * | 1,2,9 | A 61 K 31/415<br>C 07 D 233/52 |
| P,A | EP - A2 - 0 049 797 (BAYER AG)<br>* Ansprüche 1 bis 5 * | 3,6,7, 10 | |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 29-10-1982 | HASS |